# FASCICULE DE BREVET EUROPEEN

(11) **EP 1 734 026 B1**
(45) Date de publication et mention de la délivrance du brevet: **30.09.2009**
(21) Numéro de dépôt: 06290804.1
(22) Date de dépôt: 17.05.2006
(51) Int. Cl.: C07C 2/66, C07C 15/107, B01J 8/24

(54) **Procédé de production de phenylalcanes fonctionnant en lit mobile**
Wanderbettverfahrenverfahren zur Herstellung von Phenylalkanen
Moving bed process for producing phenylalkanes

(30) Priorité: 09.06.2005 FR 0505993
(43) Date de publication de la demande: 20.12.2006
(73) Titulaire: Institut Français du Pétrole, 92852 Rueil Malmaison Cedex (FR)
(72) Inventeur: Guillon, Emmanuelle, 69390 Vernaison (FR); Sanchez, Eric, 92500 Rueil Malmaison (FR)

(56) Documents cités:
- EP-A- 0 765 849
- US-A- 4 008 291
- US-A- 4 301 317
- US-A- 4 973 780
- US-A- 5 453 553
- US-A- 5 675 048

## Description

### Domaine technique

La présente invention concerne un procédé de production de phénylalcanes par alkylation catalytique d'un composé aromatique au moyen d'hydrocarbures oléfiniques comprenant en général de 9 à 16 atomes, et préférentiellement de 10 à 14 atomes de carbone par molécule.

Les phénylalcanes obtenus selon le procédé de l'invention constituent des précurseurs de choix pour la formulation de détergents, et en particulier de certains détergents biodégradables, par exemple après sulfonation.

Actuellement, les bases pour détergents biodégradables font largement appel aux alkylbenzènes ou phénylalcanes. La production de ce type de composés est en croissance régulière. Une des propriétés principales recherchées pour ces composés est leur biodégradabilité qui nécessite que les dits composés soient des alkylbenzènes linéaires (LAB) ou légèrement branchés (MAB), selon la définition du brevet US6187981.

Les alkylbenzènes sont généralement obtenus par alkylation du benzène au moyen d'oléfines ayant généralement de 9 à 16 atomes de carbone.

### Art antérieur

Les procédés d'alkylation du benzène les plus connus utilisent de l'acide fluorhydrique comme catalyseur acide. Ce procédé conduit à la formation des isomères 2-, 3-, 4-, 5- et 6-phénylalcanes. Le principal inconvénient de ce procédé est lié aux contraintes d'environnement car il pose des problèmes de sécurité sévères d'une part, et de retraitement de déchets d'autre part.

De plus, la séparation du catalyseur des produits de la réaction est difficile à mettre en oeuvre. Pour résoudre ces inconvénients, il a été proposé un procédé d'alkylation du benzène par des oléfines linéaires en présence d'un catalyseur solide acide.

Les catalyseurs solides acides constituent une alternative intéressante à l'emploi des catalyseurs acides antérieurs. Toutefois, l'inconvénient majeur des catalyseurs solides acides est leur rapide désactivation au cours de la réaction d'alkylation par adsorption à la surface desdits catalyseurs d'espèces hydrocarbonées lourdes, souvent désignées globalement par l'homme du métier comme du coke.

Plusieurs brevets ont déjà enseigné des solutions en vue de limiter, voire d'empêcher la désactivation des catalyseurs solides acides d'alkylation.

Par exemple, pour une technologie en lit fixe, le brevet US 2,541,044 revendique un procédé continu d'alkylation mettant en oeuvre plusieurs réacteurs fonctionnant en parallèle avec basculement périodique de chaque réacteur de la phase de réaction d'alkylation à une phase de réjuvénation du catalyseur se déroulant à haute température au moyen d'un flux d'hydrocarbure alkylé.

De manière similaire, le brevet US 5,648,579 divulgue un procédé continu d'alkylation en présence d'un catalyseur solide acide dont le niveau d'activité est maintenu en procédant alternativement à une étape de réaction d'alkylation (benzène + oléfines) et à une étape de lavage au benzène durant laquelle le flux d'oléfines est interrompu pour une durée de cycle de 10 minutes à 1 heure.

Le brevet US 5,453,553 divulgue un procédé pour la production d'alkylbenzènes linéaires en présence d'hydrogène avec utilisation d'un catalyseur solide comprenant une phase métallique en contact étroit avec une zéolithe.

Les procédés antérieurs divulgués dans US 5,648,579 et US 5,453,553 nécessitent d'arrêter la production d'alkylbenzènes pendant le temps nécessaire à la phase de réjuvénation du catalyseur.

Plusieurs brevets revendiquent l'utilisation de procédés continus pour l'alkylation des aromatiques, c'est à dire de procédés dans lesquels le catalyseur solide est en mouvement et circule d'une zone d'alkylation à une zone de régénération.
- Les brevet US 5675048 et US 5789640 revendiquent un procédé d'alkylation utilisant un réacteur en lit fluidisé en phase liquide avec une zone de séparation catalyseur / hydrocarbures et une ou plusieurs zones de régénération. La technologie en lit fluidisé est très différente de celle en lit mobile dans la mesure où elle nécessite une séparation du catalyseur et des effluents de la réaction qui n'existe pas dans la technologie lit mobile.
- Le brevet US 4973780 revendique un procédé d'alkylation du benzène par des oléfines en lit mobile tel qu'une partie du catalyseur désactivé est remplacé périodiquement.
   Le catalyseur est régénéré par combustion du coke en présence d'oxygène. Un tel procédé conduit à des durées de vie du catalyseur relativement faible compte tenu de la dégradation des propriétés physico-chimiques du catalyseur soumis à de nombreux cycles de combustion. Plusieurs brevets revendiquent l'utilisation de procédés continus pour l'alkylation des aromatiques, c'est à dire de procédés dans lesquels le catalyseur solide est en mouvement et circule d'une zone d'alkylation à une zone de régénération.
- Les brevet US 5675048 et US 5789640 revendiquent un procédé d'alkylation utilisant un réacteur en lit fluidisé en phase liquide avec une zone de séparation catalyseur / hydrocarbures et une ou plusieurs zones de régénération. La technologie en lit fluidisé est très différente de celle en lit mobile dans la mesure où elle nécessite une séparation du catalyseur et des effluents de la réaction qui n'existe pas dans la technologie lit mobile.
- Le brevet US 4973780 revendique un procédé d'alkylation du benzène par des oléfines en lit mobile tel qu'une partie du catalyseur désactivé est remplacé périodiquement.
   Le catalyseur est régénéré par combustion du coke en présence d'oxygène. Un tel procédé conduit à des durées de vie du catalyseur relativement faible compte tenu de la dégradation des propriétés physico-chimiques du catalyseur soumis à de nombreux cycles de combustion. Dans la présente invention le cycle de régénération est couplé au cycle de réjuvénation, de sorte que la régénération est utilisée avec une fréquence beaucoup plus limitée.
   De plus, ce brevet est orienté vers la production d'ethylbenzene ou de cumène qui nécessite des conditions opératoires très différentes de celles de la présente invention.
- Les brevets US4008291 / 4028430 / 4049739 / 4072729 et US 5523503 revendiquent l'utilisation d'un procédé à co ou contre courant simulé pour l'alkylation des aromatiques. Dans les procédés en lit mobile simulé, le catalyseur solide est immobile et les points d'injection de la charge et de soutirage des effluents varient au cours du temps de sorte que le comportement d'un tel réacteur est équivalent à celui d'une circulation du solide catalytique. Il s'agit d'une technologie qui s'apparente davantage aux lits fixes. De plus la gestion des points d'injection et de soutirage variables au cours du temps, est une complication importante.

### Description sommaire de l'invention

Le procédé de production de phenylalcanes en continu selon la présente invention, fait appel à au mois un réacteur opérant en lit mobile, le dit réacteur étant divisé en plusieurs zones réactionnelles parcourues en série par la charge et les effluents, chaque zone pouvant fonctionner avec un catalyseur identique ou différent, et le dit catalyseur étant envoyé en continu dans un circuit de réjuvénation, et de manière séquentielle dans un circuit de régénération.

La nouveauté de la présente invention réside dans l'application de la technologie en lit mobile à la réaction d'alkylation d'un composé aromatique, le réacteur utilisé étant divisé en plusieurs zones réactionnelles avec étagement de l'alimentation dans chacune des zones réactionnelles, et chaque zone disposant d'un circuit de réjuvénation et d'un circuit de régénération, les dits circuits pouvant dans certains cas être communs à plusieurs zones réactionnelles.

Dans les procédés en lit mobile, le catalyseur se trouve généralement sous forme de particules approximativement sphériques, de taille de l'ordre du millimètre, et parcourt le réacteur en écoulement descendant à des vitesses linéaires de l'ordre du mètre par heure.

Typiquement, les procédés en lit mobile du type reformage catalytique font appel à un ou plusieurs réacteurs fonctionnant en série avec un circuit de régénération du catalyseur commune à l'ensemble des réacteurs. Le terme série signifie que chaque réacteur de la série est alimenté par le catalyseur usé issu du réacteur précédent.

Le réacteur selon l'invention est divisé en plusieurs zones réactionnelles comportant chacune un catalyseur qui peut être identique ou différent d'une zone à l'autre.

De manière générale, le catalyseur d'une zone donnée est prélevé en continu en sortie de la dite zone pour être introduit dans le circuit de réjuvénation, puis après réjuvénation il est réintroduit en tête de la zone d'où il a été prélevé.

A certaines périodes de temps, le catalyseur de la zone considérée est envoyé dans le circuit de régénération, puis le catalyseur régénéré est réintroduit en tête de la zone d'où il a été prélevé.

De manière préférée, le circuit de réjuvénation sera commun pour les différentes zones réactionnelles. Cependant, chaque zone réactionnelle peut éventuellement posséder son propre circuit de réjuvénation. C'est en particulier le cas pour les zones réactionnelles ayant des catalyseurs différents. C'est l'homme du métier qui selon les contraintes économiques ou la facilité d'opération choisira la meilleure configuration possible du procédé en terme de décomposition du réacteur en plusieurs zones réactionnelles, de circuit de réjuvénation et de circuit de régénération.

Le circuit de régénération peut être commun à plusieurs zones dans la mesure où la régénération du catalyseur de plusieurs zones n'est généralement pas réalisée simultanément.

Dans la configuration générale, le catalyseur d'une zone ne circule pas dans une autre zone. Mais une configuration dans laquelle un certain nombre de zones sont parcourues en série par le même catalyseur reste parfaitement dans le cadre de l'invention. Dans ce cas, le sous-ensemble des zones parcourues en série par le même catalyseur possède un circuit de réjuvénation commun à l'ensemble des dites zones, le catalyseur étant prélevé en continu en sortie de la dernière zone, puis après réjuvénation étant réintroduit en tête de la première zone de la dite série.

On parlera dans la suite du texte de sous série pour désigner lorsqu'il existe, le sous-ensemble des zones réactionnelles parcourues en série par le même catalyseur.

Cette sous série n'existe pas toujours, le réacteur selon l'invention pouvant ne comporter que des zones réactionnelles sans circulation d'une zone à l'autre.

Le procédé de production de phényl alcanes par alkylation d'un composé aromatique selon l'invention peut donc être défini de la manière la plus générale comme formé d'une série de zones réactionnelles dans laquelle on peut distinguer deux sous-ensembles:
- le sous-ensemble des zones réactionnelles fonctionnant avec un catalyseur qui ne circule pas vers une autre zone et qui possèdent donc leur propre circuit de réjuvénation. Le catalyseur d'une telle zone réactionnelle est prélevé en sortie de chacune des zones, introduit en continu dans le circuit de réjuvénation, et réintroduit après réjuvénation en tête de la dite zone.
- le sous-ensemble des zones réactionnelles parcourues en série par le catalyseur. Pour ce sous-ensemble, le catalyseur prélevé en sortie de la dernière zone de la sous série est introduit en continu dans le circuit de réjuvénation, et réintroduit en tête de la première zone de la dite sous série.
   La charge d'une zone réactionnelle de rang n est constituée des effluents de la zone réactionnelle de rang n-1, auxquels s'ajoute une fraction de la charge oléfinique, et éventuellement, lorsque celui ci est introduit de manière fractionnée, une fraction du composé aromatique à alkyler.
   De manière préférée, le composé aromatique à alkyler est introduit en intégralité dans la première zone réactionnelle, mais une introduction fractionnée du dit composé aromatique dans chacune ou une partie des zones réactionnelles, reste parfaitement dans le cadre de l'invention.
   La charge oléfinique est toujours introduite de manière fractionnée dans chacune des zones réactionnelles, en respectant un certain ratio par rapport à la quantité de composé aromatique totale.

### Description détaillée de l'invention

La présente invention décrit un procédé de production de phénylalcanes en lit mobile faisant appel à au moins un réacteur divisé en plusieurs zones réactionnelles, avec étagement de l'introduction de la charge oléfinique dans chacune des zones réactionnelles, et réactivation du catalyseur au moyen d'un circuit de réjuvénation fonctionnant en continu, et d'un circuit de régénération fonctionnant de manière séquentielle.

Dans certains cas extrêmes, la régénération pourrait également se faire de manière continue tout en restant dans le cadre de l'invention, mais du fait de l'existence d'un circuit de réjuvénation fonctionnant en continu, le circuit de régénération fonctionnera dans la grande majorité des cas de manière séquentielle, à une fréquence optimisée.

Le pilotage judicieux de la fréquence de la régénération permet d'augmenter de façon très sensible la durée de vie du catalyseur et fait partie intégrante de l'invention.

La présente invention décrit en outre un procédé de production de phénylalcanes dont la sélectivité en produits linéaires ou branchés, et de façon plus générale, la concentration en isomère 2-phenyl, peut être contrôlée par un choix approprié du catalyseur utilisé dans chaque zone réactionnelle.

La présente invention consiste donc en un procédé de production de phénylalcanes par alkylation d'un composé aromatique, par au moins une oléfine ayant de 9 à 16 atomes de carbone par molécule, et préférentiellement ayant de 10 à 14 atomes de carbone par molécule, ladite réaction étant mise en oeuvre dans au moins un réacteur divisé en plusieurs zones réactionnelles, chaque zone réactionnelle fonctionnant en lit mobile avec un catalyseur solide acide.

De manière préférée dans le cadre de la production de phénylalcanes, la charge aromatique est introduite entièrement en tête de la première zone réactionnelle, et la charge oléfinique est introduite sous forme de fractions dans chacune des zones réactionnelles.

Les oléfines utilisées comme agent alkylant dans le ou les réacteurs catalytiques d'alkylation selon l'invention contiennent de préférence de 10 à 14 atomes de carbone par molécule.

De préférence, les oléfines utilisées pour la réaction d'alkylation sont majoritairement linéaires, c'est à dire que préférentiellement les oléfines linéaires représentent au moins 50% poids de la charge oléfinique entrant dans chaque zone réactionnelle et de manière préférée au moins 60% poids de ladite charge. Dans le cas où la charge oléfinique provient d'un effluent d'unité de déshydrogénation de n-paraffines, celle-ci peut contenir jusqu'à 20% poids de composés aromatiques lourds formés dans l'unité de déshydrogénation, en plus des paraffines linéaires non converties et des oléfines.

Le composé aromatique utilisé comme réactif dans le ou les réacteurs catalytiques d'alkylation est de préférence le benzène

Les différentes zones réactionnelles fonctionnent en série du point de vue charge/ effluent, en ce sens que l'alimentation de la zone réactionnelle n est constituée de la fraction de charge oléfinique d'alkylation, des effluents de la zone réactionnelle n-1, et éventuellement d'une fraction de composé aromatique à alkyler.

De manière générale, le catalyseur de chaque zone réactionnelle est prélevé en sortie de la dite zone, dirigé en continu vers une zone de réjuvénation, puis est réintroduit en tête de la dite zone réactionnelle.

Certaines zones réactionnelles formant une sous série, sont parcourues en série par le même catalyseur, le dit catalyseur étant alors prélevé en sortie de la dernière zone de la sous série, introduit dans le circuit de réjuvénation commun aux zones de la dite sous série, et réintroduit après réjuvénation en tête de la première zone réactionnelle de la dite sous série.

Selon un mode de réalisation de l'invention, de manière séquentielle, le catalyseur de chaque zone réactionnelle est prélevé en sortie de la dite zone, introduit dans le circuit de régénération, passe généralement ensuite dans le circuit de réjuvénation, et est réintroduit en tête de la zone réactionnelle considérée.

Le circuit de réjuvénation est caractérisé par un lavage du catalyseur au moyen d'un flux d'hydrocarbures aromatiques, généralement du benzène.

Le circuit de régénération est caractérisé par une combustion contrôlée des composés hydrocarbures adsorbés à la surface du catalyseur.

Dans la suite du texte, on parlera de manière générale de réactivation pour désigner l'un ou l'autre des circuits de réjuvénation ou de régénération.
- Plus précisément, la réjuvénation consiste à laver le catalyseur solide partiellement désactivé par un composé aromatique porté à une température généralement supérieure à 100°C.
   De manière avantageuse, ledit composé aromatique est du benzène.
   La réjuvénation est réalisée après avoir transporté le catalyseur au moyen d'une conduite de transport appelée "lift liquide" vers un ballon dit ballon de réjuvénation, dans lequel le catalyseur solide acide est mis en contact avec le composé aromatique permettant le lavage du catalyseur, à une température habituellement supérieure à la température de l'alkylation.
   La température à laquelle est réalisée le réjuvénation est généralement supérieure à 100°C, de manière préférée supérieure à 150°C, et de manière encore préférée supérieure à 200°C.
   La réjuvénation est effectuée pendant une durée suffisante, avantageusement de l'ordre de quelques heures, de façon à garantir l'élimination complète des hydrocarbures adsorbés sur les sites actifs du catalyseur solide.
- La régénération comprend typiquement une phase de combustion ménagée des dépôts carbonés formés sur le catalyseur, par exemple à l'aide d'un mélange air/azote, ou d'air appauvri en oxygène ou simplement d'air, préférentiellement déshydraté, et peut éventuellement comprendre d'autres phases complémentaires de traitement du catalyseur.

La zone de régénération peut être opérée également en lit mobile ou en lit fixe, à une pression généralement voisine de la pression moyenne du procédé et à une température généralement comprise entre 400°C à 650 °C.

La zone de régénération peut éventuellement être opérée à une pression plus faible moyennant dans ce cas un ballon tampon entre le séparateur et le régénérateur.

Le catalyseur ayant subi une réactivation, par réjuvénation et/ou par régénération, est alors transféré par lift ou par écoulement gravitaire en fonction de la géométrie de l'installation, vers l'entrée de la zone réactionnelle dans laquelle il travaille, ou à l'entrée de la série de zones réactionnelles si plusieurs zones réactionnelles sont concernées par le même catalyseur.

Le catalyseur peut circuler à co-courant d'ensemble par rapport à la charge, ou à courant croisé. Dans ce dernier cas, la charge est introduite à la périphérie de la zone réactionnelle considérée, puis est collectée dans un puit central situé approximativement au centre de la dite zone réactionnelle. On pourra, pour obtenir d'autres détails sur les procédés en lit mobile, se référer notamment aux brevets US 3,838,039 , US 5,336,829 , US 5,849,976, et à la demande de brevet EP 1 195 424A1. Une circulation inverse de la charge, du centre du réacteur vers la périphérie est également possible.

Du fait de la réactivation en continu du catalyseur, les performances catalytiques sont maintenues à l'optimum durant de très longues périodes de fonctionnement, et il n'y a plus de notion de durée de cycle comme dans les procédés issus de l'art antérieur.

De plus, le procédé selon l'invention présente également l'avantage de produire des phénylalcanes dont la sélectivité en produits linéaires, c'est-à-dire ne présentant pas de ramifications sur la chaîne alkyle portée par le groupe benzénique, et particulièrement la sélectivité en 2-phenyl, est contrôlable, ce qui est intéressant car les spécifications des produits recherchés pour une application dans la formulation des détergents sont "très variables".

Un autre avantage de l'invention est l'optimisation de la quantité de charge oléfinique à introduire dans les différentes zones réactionnelles du ou des réacteur(s) catalytique(s).

En effet, contrairement aux procédés antérieurs dans lesquels la totalité de la quantité d'oléfines est généralement introduite en une seule fois, le procédé selon l'invention permet par le fractionnement de la charge oléfinique, de réduire la quantité de benzène nécessaire par rapport aux procédés antérieurs, tout en maintenant un même rapport composé(s) aromatique(s)/oléfines, de préférence benzène/oléfines, dans le ou les réacteurs catalytiques.

Cette possibilité de moduler le rapport aromatique /oléfines représente une économie au niveau de l'investissement initial, du coût opératoire, et permet, en combinaison avec la réjuvénation continue du catalyseur, d'augmenter considérablement la durée de vie du dit catalyseur.

De plus, contrairement à certains procédés antérieurs, le procédé selon l'invention ne nécessite pas l'élimination, en amont du ou des réacteur(s) d'alkylation, des composés précurseurs de coke responsables de la désactivation, c'est à dire essentiellement les composés aromatiques lourds issus par exemple d'une unité de déshydrogénation.

En effet, la réjuvénation du catalyseur en continu permet de tolérer ces composés aromatiques lourds dans la charge d'alkylation jusqu'à des teneurs de 80% dans la charge oléfinique, le dit pourcentage étant rapporté aux seuls composés oléfiniques et aromatiques (c'est à dire en excluant les autres composés présents dans la charge d'alkylation, tels que les normales paraffines).

Il est bien clair pour l'homme du métier que la fréquence de la régénération, sera principalement conditionnée par la teneur en ces composés aromatiques, précurseurs de la désactivation, dans la charge d'alkylation.

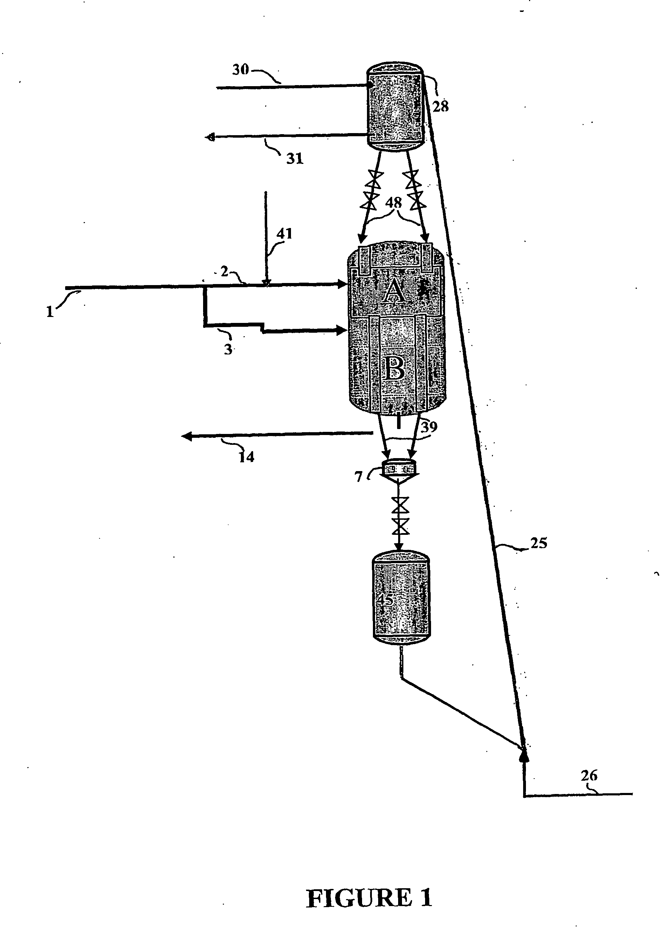

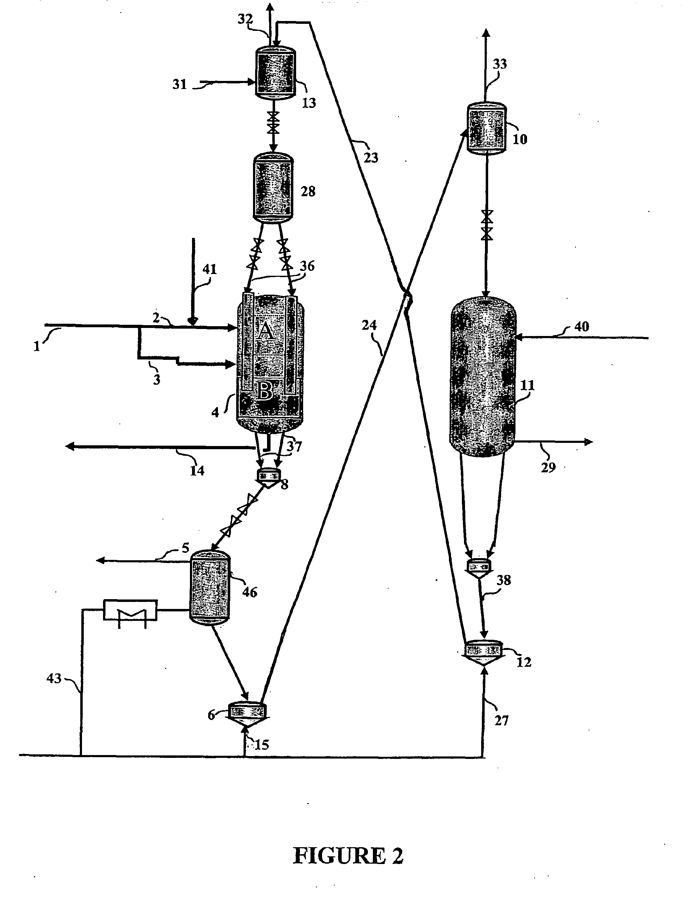

La suite de la description sera mieux comprise en suivant les figures 1 et 2 sur lesquelles sont représentés respectivement le circuit de la charge et des effluents, le circuit du catalyseur pour le circuit de réjuvénation (figure 1), et le circuit du catalyseur pour le circuit de régénération (figure 2) avec un nombre de zones réactionnelles n égal à 2.

Sur ces figures on a représenté deux zones réactionnelles, notées A et B, sans circulation de catalyseur de l'une vers l'autre.

La décomposition en 2 figures est uniquement faite pour la clarté de la compréhension, mais le procédé selon l'invention comprend bien un circuit de réjuvénation en continu, et un circuit de régénération séquentiel.

L'installation permettant de réaliser le procédé de production de phénylalcanes selon l'invention comprend donc au moins un réacteur catalytique d'alkylation (4), un ballon de réjuvénation (28) et un ballon de régénération (11), appelée dans la suite régénérateur (11).

Le réacteur (4) est divisé en deux zones réactionnelles, une zone réactionnelle supérieure (A) et une zone réactionnelle inférieure (B), munie chacune de moyens d'introduction du catalyseur (48) et de soutirage (39) pour la zone réactionnelle (A) située en partie supérieure du réacteur (figure 1), et de moyens de d'introduction (36) et de soutirage (37) du catalyseur pour la zone réactionnelle (B) située en partie inférieure du réacteur (4) (figure 2). Les moyens de soutirage comprennent en outre au moins un réservoir (45) alimenté à partir d'un pot intermédiaire (7) permettant de diriger le catalyseur vers le circuit de réjuvénation, et un réservoir (46) alimenté à partir d'un pot intermédiaire (8) permettant de diriger le catalyseur vers le circuit de régénération. Les pots intermédiaires (7) et (8) pourront éventuellement être confondus.

La charge oléfinique (1) est divisée en une fraction (2) alimentant la zone réactionnelle A, et une fraction (3) alimentant la zone réactionnelle B.

La fraction de charge oléfinique (2) est mélangée avec le composé aromatique introduit intégralement par la ligne (41) pour constituer la charge de la zone réactionnelle A.

La fraction de charge oléfinique (3) est mélangée avec les effluents de la zone réactionnelle A (non représentés sur les figures) pour constituer la charge de la zone réactionnelle B.

Les effluents réactionnels de la zone réactionnelle B sont évacués de la dite zone par la ligne (14) où ils sont dirigés vers une zone de fractionnement non représentée sur les figures 1 et 2.

Le nombre n de zones réactionnelles de chaque réacteur est compris entre 1 et 10, et de manière préférée compris entre 1 et 5.

On introduit, à l'entrée de chacune des zones réactionnelles une fraction de la quantité totale d'oléfines nécessaire à la réaction d'alkylation du composé aromatique, de préférence le benzène, de manière que le rapport du poids d'aromatique à alkyler sur le poids d'oléfines à l'entrée de chaque zone réactionnelle soit compris entre 30 et 100, et préférentiellement compris entre 30 et 70.

Il est également très avantageux pour un réacteur catalytique comportant n zones réactionnelles, que la fraction d'oléfines introduite dans chacune des zones réactionnelles représente sensiblement 1/n de la quantité totale d'oléfines nécessaire à la réaction d'alkylation.

Selon un autre mode de fonctionnement, la fraction d'oléfines introduite à l'entrée de chacune des zones réactionnelles est telle que le rapport pondéral aromatique à alkyler sur oléfines est étagé dans le sens croissant ou décroissant, dans chacune desdites zones réactionnelles. C'est à dire que ce rapport peut être augmenté (ou diminué) selon une certaine progression, lorsqu'on passe de la zone réactionnelle de rang n à la zone réactionnelle de rang n+1 pour des raisons de qualité de produits ou de stabilité du catalyseur.

Le rapport précédemment mentionné peut également être modifié en ajustant la fraction du composé aromatique à alkyler lorsque celui ci est introduit de manière fractionné.

Chaque fraction de charge oléfinique est généralement contenue dans une charge hydrocarbonée contenant principalement des paraffines. Les paraffines contenues dans ladite charge sont des paraffines en C₉-C₁₆, de préférence en C₁₀-C₁₄. Les oléfines représentent généralement de 5 à 100% poids de la charge hydrocarbonée introduite à l'entrée de chacune des zones réactionnelles.

Chacune des zones réactionnelles est opérée à une température inférieure à 400°C, généralement comprise entre 50°C et 350°C, de manière préférée comprise entre 70°C et 300°C, et de manière encore préférée comprise entre 80°C et 250°C.

Chaque zone réactionnelle fonctionne sous une pression de 1 à 10 MPa (Méga Pascal, 1 MPa = 10 bars), de préférence sous une pression de 1 à 7 MPa, avec un débit d'hydrocarbures liquides dont la vitesse spatiale est d'environ 0,1 à 80, et de préférence de 0,5 à 50 volumes par volume de catalyseur et par heure.

La réaction d'alkylation mise en oeuvre dans le ou les réacteurs catalytiques d'alkylation selon l'invention est généralement suivie d'au moins une étape de séparation afin de récupérer les réactifs en excès d'une part, et les phénylalcanes recherchés d'autre part.

Plus précisément, à la sortie du réacteur catalytique d'alkylation, on fractionne en général les effluents du dit réacteur évacués par la ligne (14) de manière à recueillir séparément
a) un premier effluent renfermant le composé aromatique, de préférence le benzène, non converti,
b) un deuxième effluent renfermant au moins une oléfine linéaire C₉-C₁₆, de préférence C₁₀₋C₁₄, non convertie, ainsi que les paraffines et éventuellement les composés aromatiques,
c) un troisième effluent renfermant des 2-, 3-, 4-, 5- et 6-phénylalcanes, et
d) un quatrième effluent renfermant au moins un poly-alkylbenzène (ou fraction poly-alkylbenzène), celui-ci pouvant être éventuellement, au moins en partie, recyclé vers une des zones réactionnelles, où il réagit avec le composé aromatique, de préférence le benzène, au contact du catalyseur solide acide contenu dans la dite zone réactionnelle, afin d'être au moins en partie transalkylé (selon une réaction de transalkylation), et on recueille un mélange de 2-, 3-, 4-, 5- et 6-phénylalcanes.

### Description du circuit de réjuvénation (figure 1)

- La réjuvénation consiste à transférer le catalyseur prélevé en sortie d'une zone réactionnelle, (la zone A dans le cas de la figure 1) vers le réservoir (45) au moyen des jambes (39).

A partir du réservoir (45), le catalyseur est transporté par un lift liquide (25) alimenté par un flux liquide (26) (un lift gazeux étant également possible moyennant dans ce cas un drainage du ballon (45)), vers un ballon de réjuvénation (28) situé par exemple au dessus du réacteur d'alkylation (4), puis à laver ledit catalyseur au moins partiellement désactivé, par un composé aromatique introduit par la ligne (30).

Les effluents de lavage contenant le composé aromatique de lavage et les hydrocarbures adsorbés à la surface du catalyseur sont évacués du ballon de réjuvénation (28) par la ligne (31). Le catalyseur réjuvéné est réintroduit en tête de la zone réactionnelle A au moyen des jambes (48).

De manière avantageuse, ledit composé aromatique est du benzène. La réjuvénation est réalisée en l'absence d'hydrocarbures oléfiniques puisque, généralement, le catalyseur avant transport subit une étape de séparation des effluents de réaction au niveau du ballon (45).

La température à laquelle est réalisée le réjuvénation est généralement supérieure à 100°C, de manière préférée supérieure à 150°C, et de manière encore préférée supérieure à 200°C.

La réjuvénation est effectuée pendant une durée suffisante, de façon à garantir l'élimination complète des hydrocarbures adsorbés dans les sites actifs de chacun des catalyseurs solides acides. Cette durée dépend du niveau de désactivation du catalyseur, mais aussi des conditions hydrodynamiques utilisées dans le ballon de réjuvénation (28) lors du lavage, en particulier de la vitesse linéaire de l'agent de lavage qui sera généralement supérieure à 1 m/s.

Le catalyseur soutiré en continu du ballon de réjuvénation (28) au moyen des jambes de descente (48) est envoyé, généralement par gravité, vers la zone réactionnelle à partir de laquelle il a été prélevé, (la zone A sur la figure1).

Dans le cas d'un catalyseur prélevé en sortie d'une zone réactionnelle appartenant à une sous série (au sens donné à ce terme dans le paragraphe description sommaire de l'invention), le catalyseur réjuvéné est réintroduit en tête de la première zone réactionnelle de la dite sous série.

### Description du circuit de régénération (figure 2)

La régénération consiste à transférer le catalyseur depuis la zone réactionnelle (notée B sur la figure 2) vers un ballon (46) par l'intermédiaire des jambes (37) et du pot intermédiaire (8), puis à transférer le catalyseur du ballon (46) vers le pot élévateur (6) à partir lequel il est transporté au moyen du gaz de transport (15) vers un ballon séparateur (10) à partir duquel il est transféré par gravité vers le régénérateur (11).

Le ballon séparateur (10) permet la séparation du catalyseur et du gaz de transport, et l'élimination des éventuelles fines particules formées par attrition au cours du dit transport. A l'intérieur du régénérateur (11), le catalyseur subit une combustion contrôlée des espèces hydrocarbonées adsorbées à sa surface, au moyen d'un gaz de combustion introduit par la ligne (40). Les effluents de combustion sont évacués par la ligne (29) vers un circuit de régénération spécifique connu de l'homme du métier.

Le catalyseur régénéré est transféré dans un pot intermédiaire (38), puis du pot intermédiaire (38), il est repris dans le pot de transport (12), alimenté par le gaz de transport (27) pour être ramené dans un ballon séparateur (13) à l'intérieur duquel le catalyseur est séparé du gaz de transport et des fines particules éventuellement formées au cours du dit transport.

De manière préférée, le catalyseur régénéré en sortie du ballon séparateur (13) est réintroduit dans le ballon de réjuvénation (28) avant d'être ramené par l'intermédiaire des jambes (36) dans la zone réactionnelle B.

Une configuration dans laquelle le catalyseur régénéré serait directement réintroduit dans la zone réactionnelle B à partir du ballon séparateur (13) resterait néanmoins dans le cadre de l'invention.

La zone réactionnelle dans laquelle est réintroduit le catalyseur régénéré peut être soit la zone réactionnelle à partir de laquelle il a été prélevé, soit la première zone réactionnelle d'une sous série de zones réactionnelles utilisant le même catalyseur au sens défini dans le paragraphe description sommaire de l'invention.

La régénération est réalisée par combustion des espèces hydrocarbonées adsorbées sur les sites actifs du catalyseur.

Plus précisément, la régénération du catalyseur par combustion comprend:
- éventuellement une première étape généralement réalisée dans un ballon (46) avant le transport du catalyseur vers le régénérateur, consistant à chauffer sous gaz inerte (43), à une température supérieure à 100°C, et de préférence comprise entre 150°C et 250 °C, et de manière encore préférée comprise entre 200°C et 300°C, ledit catalyseur désactivé, de façon à éliminer les hydrocarbures liquides adsorbés par la ligne (5), et
- une deuxième étape consistant à mettre en contact dans le ballon de régénération (11) le catalyseur avec un gaz contenant de l'oxygène (40), en augmentant progressivement la température jusqu'à ce que la réaction exothermique de combustion du coke soit observée, en général entre 300°C et 600°C, et préférentiellement entre 400°C et 500°C.

La première étape de chauffage sous gaz inerte permet d'éviter tout risque de montée en température non contrôlée durant la deuxième étape qui correspond à la combustion proprement dite du coke.

La majorité du coke est brûlée au cours de l'étape de combustion.

La teneur pondérale en coke résiduel sur le catalyseur après combustion est généralement inférieure à 20%, et de préférence inférieure à 10% de la teneur en coke sur le catalyseur avant la dite combustion.

Le gaz contenant de l'oxygène utilisé au cours de l'étape de combustion est généralement un mélange d'oxygène et de gaz inerte, contenant avantageusement de 0,1% à 20% en volume d'oxygène, et de préférence de 0,2% à 10% en volume d'oxygène.

Il peut s'agir par exemple d'air, ou d'air dilué dans un gaz inerte.

La régénération est déclenchée à des périodes de temps déterminées à partir d'indicateurs généralement placés en ligne, et qui, directement ou indirectement, reflètent le degré de désactivation du catalyseur.

Parmi les indicateurs bien connus de l'homme du métier, on peut citer la conversion en oléfines introduites dans chaque zone réactionnelle, le niveau de coke déposé sur le catalyseur, ou bien encore la température opératoire dans les zones réactionnelles, ou encore et de manière préférée, la différence de température entre la sortie et l'entrée de chaque zone réactionnelle qui généralement sera maintenue à une certaine valeur.

Plus précisément, dans une configuration préférée, le réacteur pour la production de phényl alcanes par alkylation d'un composé aromatique par une charge oléfinique selon l'invention est constitué d'une série de n zones réactionnelles, chaque zone réactionnelle possédant au moins un thermocouple de mesure de la température en entrée et en sortie de la dite zone, et la séquence de régénération du catalyseur contenu dans la dite zone étant déclenchée lorsque la différence de température entre la sortie et l'entrée de la dite zone dépasse une certaine valeur.

Les catalyseurs utilisés dans le procédé selon l'invention sont tous types de solides acides capables de réaliser la réaction d'alkylation. Il peut s'agir de solides acides amorphes ou cristallisés.
- Parmi les catalyseurs amorphes, les silices alumines, alumines, dopés ou non par des halogènes sont préférés.
- Parmi les catalyseurs cristallisés, les zéolithes de structure cristalline, par exemple ayant une structure telle que définie dans la Classification "Atlas of Zeolite Framework Type" (Atlas des différents types de structure des géolithes), (W. M Meier, D. H. Olson and Ch. Baerlocher, 5th revised edition, 2001, Elsevier). sont préférés.

De préférence, le catalyseur comprend au moins une zéolithe choisie dans le groupe constitué par les zéolithes de type structural FAU, MOR, MTW, OFF, MAZ, BEA EUO, UTL et NES.

De manière préférée, le catalyseur contenu dans chacune, ou une partie au moins des zones réactionnelles, comprend au moins une zéolithe Y, avantageusement une zéolithe Y désaluminée, de rapport atomique Si/AI global supérieur à 4, de préférence compris entre 8 et 100, et de manière encore préférée compris entre 15 et 70.

Selon un autre mode de réalisation de la présente invention, il peut être avantageux d'utiliser comme catalyseur solide acide dans chacune des zones réactionnelles, un mélange de catalyseurs. Il peut s'agir par exemple d'un mélange d'un catalyseur cristallisé et d'un catalyseur amorphe. Il peut s'agir également d'un mélange de zéolithes constitué d'au moins une zéolithe Y telle que décrite précédemment, et d'au moins une zéolithe de type structural MOR, notamment une zéolithe mordénite.

Le procédé de production de phényl alcanes par alkylation d'un composé aromatique selon l'invention peut contenir dans certaines des zones réactionnelles un catalyseur solide acide cristallisé de type structural choisi dans le groupe formé par FAU, MOR, MTW, OFF, MAZ, BEA EUO, UTL et NES.

Le catalyseur utilisé dans une partie au moins des zones réactionnelles peut également être un catalyseur solide amorphe choisi dans le groupe formé par les silices alumines, et les alumines dopés ou non par des composés halogènés.

Le procédé de production de phényl alcanes par alkylation d'un composé aromatique selon l'invention peut contenir dans certaines des zones réactionnelles un mélange d'au moins deux catalyseurs solides cristallisés de type structural choisi dans le groupe formé par FAU, MOR, MTW, OFF, MAZ, BEA EUO, UTL et NES.

Enfin dans certains cas le procédé de production de phénylalcanes selon l'invention pourra contenir dans certaines zones réactionnelles un mélange d'au moins un catalyseur solide cristallisé de type structural choisi dans le groupe formé par FAU, MOR, MTW, OFF, MAZ, BEA EUO, UTL, NES et d'un catalyseur solide amorphe choisi dans le groupe formé par les silices alumines, et les alumines dopés ou non par des composés halogènés.

Le mélange desdites zéolithes se trouvant à l'état de poudre, peut être réalisé par toutes les techniques de mélange de poudres connues de l'homme du métier, et suivi de la mise en forme.

Le mélange peut en particulier être réalisé avec une matrice, généralement amorphe, ou avec une poudre humide de gel d'alumine. La mise en forme peut aussi être réalisée avec d'autres matrices que l'alumine, telles que par exemple la magnésie, les silice-alumines amorphes, les argiles naturelles (kaolin, bentonite, sepiolite, attapulgite), la silice, l'oxyde de titane, l'oxyde de bore, la zircone, les phosphates d'aluminium, les phosphates de titane, les phosphates de zirconium, le charbon et leurs mélanges.

On préfère généralement utiliser des matrices contenant de l'alumine, sous toutes les formes connues de l'homme du métier, et de manière encore plus préférée contenant de l'alumine gamma.

Le catalyseur contenu dans chacune des zones réactionnelles du ou des réacteurs catalytiques d'alkylation pour la mise en oeuvre du procédé selon l'invention, est mis sous forme de grains de forme et de dimension compatibles avec les différents modes de transport utilisés dans la présente invention.

Le catalyseur devant circuler à l'intérieur des zones réactionnelles en lit mobile, est le plus souvent sous forme de billes sphériques avec un diamètre de bille de quelques millimètres, typiquement de 0,5 à 5 mm, et de manière préférée de 1 à 4 mm.

Pour la fabrication du catalyseur, on peut utiliser des techniques classiques, par exemple mélanger la zéolithe avec des précurseurs de gel de silice et/ou des gels de silice, puis mettre en forme de petites billes par coagulation de gouttes, sécher puis calciner les dites billes pour obtenir le catalyseur final à la forme et la dimension appropriées.

Après l'étape de mise en forme, le solide obtenu est soumis à une étape de séchage à une température comprise entre 100 et 300°C, de préférence entre 120 et 200°C, puis à une étape de calcination à une température comprise entre 300 et 600°C, de préférence entre 350 et 550°.

### EXEMPLE SELON L'INVENTION

L'invention sera mieux comprise à la lecture de l'exemple détaillé suivant représentatif d'un mode particulier de réalisation d'un réacteur selon l'invention.

Cet exemple n'est nullement limitatif et seulement destiné à illustrer un mode particulier de réalisation du procédé selon l'invention.

Le réacteur d'alkylation utilisé comprend 2 zones réactionnelles, une zone réactionnelle supérieure notée A, et une zone réactionnelle inférieure notée B. Il n'y a pas de circulation de catalyseur d'une zone à l'autre.

Le catalyseur utilisé dans les deux zones est constitué par 70% poids de zéolithe Y désaluminée avec un rapport Si/Al de 30.

La taille des particules sphériques de catalyseur est de 2,5 mm.

La charge oléfinique d'alkylation (1) a la composition suivante:
paraffines de C9 à C16: 85% poids
oléfines de C9 à C16: 10% poids
aromatiques de C9 à C16: 5% poids

La charge oléfinique d'alkylation (1) est divisée en une première fraction (2) introduite dans la première zone réactionnelle (A) et une seconde fraction (3) introduite dans la seconde zone réactionnelle (B).

La charge constituée de benzène (41) est mélangée avec de la charge d'alkylation (1) puis introduite dans la zone réactionnelle (A) par la conduite (2). Le rapport pondéral benzène/oléfine est égal à 60. La totalité du benzène est introduite en tête de réacteur.

L'alimentation de la zone réactionnelle (B) est constituée de l'effluent réactionnel de la zone réactionnelle (A) et du flux de charge oléfinique (3).

A la sortie de la seconde zone réactionnelle (B), l'effluent réactionnel est envoyé par la conduite 14 vers une section de séparation non représentée sur la figure 1.

Les zones réactionnelles A et B fonctionnent à une température de 120°C sous une pression de 4 MPa.

La section de séparation, non représentée, comprend plusieurs colonnes de fractionnement, et a pour but de séparer les produits de réaction recherchés, c'est à dire principalement un mélange de 2-phénylalcane, 3-phénylalcane, 4-phénylalcane, 5-phénylalcane et 6-phénylalcane qui est envoyé au stockage.

Le catalyseur des zones réactionnelles A et B est soutiré respectivement par les jambes de descente (39) et (37). Afin d'illustrer le fonctionnement typique de l'installation, on va décrire pour le catalyseur issu de la zone réactionnelle (A) le circuit de réjuvénation (voir figure 1), et pour le catalyseur issu de la zone réactionnelle (B) le circuit de régénération (voir figure 2).

Il faut cependant rappeler que le mode de fonctionnement préféré de la présente invention consiste à réjuvéner en continu le catalyseur issu de la zone réactionnelle A et B dans le même circuit de réjuvénation (les catalyseurs des zones A et B étant identiques, et soutirés en parallèle), et de façon séquentielle à régénérer le catalyseur issu des zones réactionnelles A et B dans le même circuit de régénération. La réjuvénation et la régénération coexistent en opération, chacune avec leur circuit spécifique.

### Circuit de réjuvénation (figure 1)

Le catalyseur issu de la zone réactionnelle (A) est soutiré par la jambe de descente (39) et envoyé dans un pot intermédiaire (7) puis dans le ballon (45) avant d'être transféré via un élévateur liquide (25) vers le ballon de réjuvénation (28) alimenté par du benzène à 250°C par la ligne (30).

L'effluent de lavage contenant le benzène de lavage et les produits adsorbés à la surface du catalyseur sont évacués par la ligne (31) et sont envoyés vers la section séparation. Le liquide de transport alimentant l'élévateur liquide (25) est introduit par la ligne (26).

Le catalyseur réjuvéné est réintroduit en tête de la zone réactionnelle (A) par l'intermédiaire de jambes de descente (48).

Le catalyseur réjuvéné est mis en contact dans la zone réactionnelle (A) avec le benzène à alkyler (41), et la charge oléfinique (2).

### Circuit de régénération (figure 2)

Le catalyseur issu de la zone réactionnelle (B) est soutiré par la jambe de descente (37), et envoyé dans un pot intermédiaire (8) puis dans le ballon (46) à l'intérieur duquel il subit un drainage, un préchauffage et un stripage jusqu'à une température de 300°C au moyen d'azote chaud introduit par la ligne (43).

Le catalyseur est ensuite transféré dans un pot élévateur (6) afin d'être convoyé via un lift gazeux (24) alimenté par l'azote de transport (15) vers la trémie supérieure (10) située au dessus du ballon de régénération (11).

Par écoulement gravitaire, le catalyseur est ensuite conduit vers le régénérateur (11) où il subit une combustion ménagée en présence d'air introduit par la ligne (40).

Les gaz de combustion sont évacués du régénérateur (11) par la ligne (29) et sont envoyés vers un circuit spécifique de régénération (non représenté sur la figure 2).

En sortie du régénérateur (11), le catalyseur régénéré passe dans une trémie (38) puis dans un pot élévateur (12) afin d'être convoyé dans la trémie supérieure (13) par un système de transport pneumatique (23) fonctionnant à l'azote (27).

Dans la trémie supérieure du réacteur (13), le catalyseur subit un balayage par un gaz (31) permettant d'évacuer les fines éventuellement créées au cours du transport qui sont dirigées par la ligne (32) vers un filtre à particules non représenté.

Le catalyseur est transféré dans le ballon de réjuvénation (28) ou il est mis en contact avec du benzene, avant d'être réintroduit dans la zone réactionnelle (B) du réacteur (4) au moyen des jambes de descente (36). Dans la zone réactionnelle B, le catalyseur régénéré est mis en contact avec la charge réactionnelle résultant de la charge oléfinique (3), et des effluents de la zone réactionnelle (A).

Le catalyseur s'écoule gravitairement dans la zone réactionnelle (B) et est recueilli en fond dans une trémie (46) par l'intermédiaire du pot (8).

Les caractéristiques des LAB (abréviation de alkyl benzène linéaires ou linear alkyl benzene selon la terminologie anglo-saxonne) produits sont les suivantes :
2 phényl alcane: supérieur à 25% poids
tétraline: inférieur à 0,5 % poids
densité: 0,86 kg/l
degré de linéarité: supérieur à 92%

## Revendications

1. Procédé de production de phényl alcanes par alkylation d'un composé aromatique par une charge contenant des oléfines de C9 à C16 faisant appel à au moins un réacteur divisé en plusieurs zones réactionnelles, chaque zone réactionnelle utilisant un catalyseur solide acide fonctionnant en lit mobile, le catalyseur de chaque zone réactionnelle étant de manière continue envoyé dans un circuit de réjuvénation à sa sortie de ladite zone réactionnelle, puis réintroduit en tête de ladite zone, et le catalyseur de chaque zone réactionnelle étant de manière séquentielle envoyé dans un circuit de régénération à la sortie de ladite zone réactionnelle, puis réintroduit en tête de ladite zone réactionnelle.

2. Procédé de production de phényl alcanes par alkylation d'un composé aromatique par une charge contenant des oléfines de C9 à C16 selon la revendication 1 dans lequel une partie des zones réactionnelles formant une sous série, fonctionnent avec le même catalyseur qui les parcourt successivement en lit mobile, ledit catalyseur étant introduit de façon continue dans le circuit de réjuvénation en sortie de la dernière zone de la sous série, puis après réjuvénation étant réintroduit en tête de la première zone de ladite sous série, ledit catalyseur étant envoyé de manière séquentielle dans le circuit de régénération à la sortie de la dernière zone de la sous série et, après régénération, étant réintroduit en tête de la première zone de ladite sous série.

3. Procédé de production de phényl alcanes par alkylation selon l'une quelconque des revendications 1 à 2 dans lequel la charge oléfinique d'alkylation contient préférentiellement des oléfines allant de C10 à à C14, et le composé aromatique à alkyler est le benzène.

4. Procédé de production de phényl alcanes par alkylation selon l'une quelconque des revendications 1 à 3 dans lequel la charge en oléfines est introduite de manière fractionnée dans chaque zone réactionnelle, de manière que le rapport aromatique/oléfines à l'entrée de chaque zone réactionnelle soit compris entre 30 et 100, et préférentiellement compris entre 30 et 70.

5. Procédé de production de phényl alcanes par alkylation d'un composé aromatique selon l'une quelconque des revendications 1 à 4 dans lequel le catalyseur utilisé dans une partie au moins des zones réactionnelles est un catalyseur solide acide cristallisé de type structural choisi dans le groupe formé par FAU, MOR, MTW, OFF, MAZ, BEA, EUO, UTL et NES.

6. Procédé de production de phényl alcanes par alkylation d'un composé aromatique selon l'une quelconque des revendications 1 à 4 dans lequel le catalyseur utilisé dans une partie au moins des zones réactionnelles est un catalyseur solide amorphe choisi dans le groupe formé par les silices alumines, et les alumines dopés ou non par des composés halogénés.

7. Procédé de production de phényl alcanes par alkylation d'un composé aromatique selon l'une quelconque des revendications 1 à 5 dans lequel le catalyseur utilisé dans certaines zones réactionnelles est une zéolithe Y désaluminée de rapport Si/Al compris entre 8 et 100, et préférentiellement compris entre 15 et 70.

8. Procédé de production de phényl alcanes par alkylation d'un composé aromatique selon l'une quelconque des revendications 1 à 7 dans lequel la taille des grains de catalyseur utilisé dans chacune des zones réactionnelles est comprise entre 0,5 et 5 mm, et préférentiellement comprise entre 1 et 4 mm.

9. Procédé de production de phényl alcanes par alkylation d'un composé aromatique selon l'une quelconque des revendications 1 à 8 dans lequel le catalyseur utilisé dans certaines zones réactionnelles est un mélange d'au moins deux catalyseurs solides cristallisés de type structural choisi dans le groupe formé par FAU, MOR, MTW, OFF, MAZ, BEA EUO, UTL et NES.

10. Procédé de production de phényl alcanes par alkylation d'un composé aromatique selon l'une quelconque des revendications 1 à 8 dans lequel le catalyseur utilisé dans certaines zones réactionnelles est un mélange de zéolithes, l'un des composants du mélange étant une zéolithe Y désaluminée avec un rapport Si/Al compris entre 8 et 10, et préférentiellement compris entre 15 et 70, et l'autre composant du mélange étant une zéolithe de type structural MOR, préférentiellement la mordénite.

11. Procédé de production de phényl alcanes par alkylation d'un composé aromatique selon l'une quelconque des revendications 1 à 10 dans lequel le catalyseur utilisé dans certaines zones réactionnelles est un mélange d'au moins un catalyseur solide cristallisé de type structural choisi dans le groupe formé par FAU, MOR, MTW, OFF, MAZ, BEA EUO, UTL, NES et d'un catalyseur solide amorphe choisi dans le groupe formé par les silices alumines, et les alumines dopés ou non par des composés halogénés.

12. Procédé de production de phényl alcanes par alkylation d'un composé aromatique selon l'une quelconque des revendications 1 à 11 dans lequel la teneur en composés aromatiques lourds contenus dans la charge oléfinique d'alkylation peut aller jusqu'à 80 % poids, en rapportant ce pourcentage aux oléfines et aux aromatiques seuls.

13. Procédé de production de phényl alcanes par alkylation d'un composé aromatique selon l'une quelconque des revendications 1 à 12 dans lequel la température d'alkylation est comprise entre 50°C et 350°C, et la pression est comprise entre 1 et 10 MPa.

14. Procédé de production de phényl alcanes par alkylation d'un composé aromatique selon l'une quelconque des revendications 1 à 13 dans lequel le gaz de combustion utilisé pour la régénération du catalyseur contient de 0,1 à 20 % en volume d'oxygène, et la température de combustion est comprise entre 300°C et 600°C.

15. Procédé de production de phényl alcanes par alkylation d'un composé aromatique selon l'une quelconque des revendications 1 à 14 dans lequel l'agent réjuvénant est un composé aromatique, de préférence le benzène, et la température de la réjuvénation est supérieure à 100°C.

16. Procédé de production de phényl alcanes selon la revendication 1 par alkytation d'un composé aromatique par une charge oléfinique constitué d'une série de n zones réactionnelles, n étant compris entre 1 et 10, et ladite série comprenant:
- un premier sous-ensemble de zones réactionnelles parcourues en série par le catalyseur circulant en lit mobile, le catalyseur prélevé en sortie de la dernière zone du premier sous-ensemble étant introduit en continu dans le circuit de réjuvénation, et réintroduit en tête de la première zone dudit premier sous-ensemble, et ledit catalyseur étant de manière séquentielle envoyé dans le circuit de régénération
- un second sous-ensemble constitué des zones réactionnelles fonctionnant avec un catalyseur fonctionnant en lit mobile, chaque zone réactionnelle possédant un circuit de réjuvénation propre, le catalyseur étant prélevé en sortie de chacune des zones, introduit en continu dans le circuit de réjuvénation, et réintroduit après réjuvénation en tête de la zone d'où il a été prélevé, et ledit catalyseur étant de manière séquentielle envoyé dans le circuit de régénération,
la charge d'une zone réactionnelle de rang n étant constituée des effluents de la zone réactionnelle de rang n-1, auxquels s'ajoutent une fraction de la charge oléfinique, et le composé aromatique à alkyler étant introduit à l'entrée de la première zone réactionnelle.

17. Procédé de production selon le revendication 16 dans lequel le nombre n de zones réactionnelles est compris entre 1 et 5.

## Claims

1. Process for the production of phenyl alkanes by alkylation of an aromatic compound by a feedstock that contains C9 to C16 olefins employing at least one reactor that is divided into several reaction zones, each reaction zone using an acidic solid catalyst that operates in a moving bed, whereby the catalyst of each reaction zone is continuously sent into a rejuvenation circuit upon leaving said reaction zone, then is reintroduced at the top of said zone, and whereby the catalyst of each reaction zone is sequentially sent into a regeneration circuit leaving said reaction zone, then is reintroduced at the top of said reaction zone.

2. Process for the production of phenyl alkanes by alkylation of an aromatic compound by a feedstock that contains C9 to C16 olefins according to claim 1, in which a portion of the reaction zones forming a sub-series operate with the same catalyst that travels through them successively in a moving bed, whereby said catalyst is introduced continuously in the rejuvenation circuit leaving the last zone of the sub-series, then after rejuvenation is reintroduced at the top of the first zone of said sub-series, whereby said catalyst is sent sequentially into the regeneration circuit leaving the last zone of the sub-series and, after regeneration, is reintroduced at the top of the first zone of said sub-series.

3. Process for the production of phenyl alkanes by alkylation according to any of claims 1 to 2, in which the olefinic feedstock for alkylation preferably contains olefins that range from C10 to C14, and the aromatic compound that is to be alkylated is benzene.

4. Process for the production of phenyl alkanes by alkylation according to any of claims 1 to 3, in which the olefin feedstock is introduced in a fractionated manner in each reaction zone so that the aromatic compound/olefin ratio at the input of each reaction zone is between 30 and 100, and preferably between 30 and 70.

5. Process for the production of phenyl alkanes by alkylation of an aromatic compound according to any of claims 1 to 4, in which the catalyst that is used in at least a portion of the reaction zones is a crystallized acidic solid catalyst of a structural type that is selected from the group formed by FAU, MOR, MTW, OFF, MAZ, BEA EUO, UTL and NES.

6. Process for the production of phenyl alkanes by alkyation of an aromatic compound according to any of claims 1 to 4 in which the catalyst that is used in at least a portion of the reaction zones is an amorphous solid catalyst that is selected from the group that is formed by the alumina silicas and the aluminas that may or may not be doped by halogenated compounds.

7. Process for the production of phenyl alkanes by alkylation of an aromatic compound according to any of claims 1 to 5, in which the catalyst that is used in certain reaction zones is a dealuminified Y zeolite of the Si/Al ratio of between 8 and 100 and preferably between 15 and 70.

8. Process for the production of phenyl alkanes by alkylation of an aromatic compound according to any of claims 1 to 7, in which the size of the catalyst grains used in each of the reaction zones is between 0.5 and 5 mm, and preferably between 1 and 4 mm.

9. Process for the production of phenyl alkanes by alkylation of an aromatic compound according to any of claims 1 to 8, in which the catalyst that is used in certain reaction zones is a mixture of at least two crystallized solid catalysts of a structural type that is selected from the group that is formed by FAU, MOR, MTW, OFF, MAZ, BEA EUO, UTL and NES.

10. Process for the production of phenyl alkanes by alkylation of an aromatic compound according to any of claims 1 to 8, in which the catalyst that is used in certain reaction zones is a mixture of zeolites, whereby one of the components of the mixture is a dealuminified Y zeolite with an Si/Al ratio of between 8 and 10, and preferably between 15 and 70, and whereby the other component of the mixture is an MOR structural-type zeolite, preferably mordenite.

11. Process for the production of phenyl alkanes by alkylation of an aromatic compound according to any of claims 1 to 10, in which the catalyst that is used in certain reaction zones is a mixture of at least one crystallized solid catalyst of a structural type that is selected from the group that is formed by FAU, MOR, MTW, OFF, MAZ, BEA EUO, UTL, and NES, and an amorphous solid catalyst that is selected from the group that is formed by the alumina silicas, and the aluminas that may or may not be doped by halogenated compounds.

12. Process for the production of phenyl alkanes by alkylation of an aromatic compound according to any of claims 1 to 11, in which the content of heavy aromatic compounds contained in the olefinic feedstock for alkylation can go up to 80% by weight, by relating this percentage to olefins and to aromatic compounds alone.

13. Process for the production of phenyl alkanes by alkylation of an aromatic compound according to any of claims 1 to 12, in which the alkylation temperature is between 50°C and 350°C, and the pressure is between 1 and 10 MPa.

14. Process for the production of phenyl alkanes by alkylation of an aromatic compound according to any of claims 1 to 13, in which the combustion gas that is used for the regeneration of the catalyst contains 0.1 to 20% by volume of oxygen, and the combustion temperature is between 300°C and 600°C.

15. Process for the production of phenyl alkanes by alkylation of an aromatic compound according to any of claims 1 to 14, in which the rejuvenating agent is an aromatic compound, preferably benzene, and the temperature of the rejuvenation is more than 100°C.

16. Process for the production of phenyl alkanes according to claim 1 by alkylation of an aromatic compound according to claim 1 by alkylation of an aromatic compound by an olefinic feedstock that consists of a series of n reaction zones, whereby n is between 1 and 10, whereby said series comprises
- A first subset of reaction zones through which the catalyst that circulates in a moving bed travels in succession, whereby the catalyst that is drawn leaving the last zone of the first subset is introduced continuously in the rejuvenation circuit and reintroduced at the top of the first zone of said first subset, and whereby said catalyst is sequentially sent into the regeneration circuit
- A second subset that consists of reaction zones that operate with a catalyst that operates in a moving bed, whereby each reaction zone has a suitable rejuvenation circuit, whereby the catalyst is drawn leaving each of the zones, introduced continuously into the rejuvenation circuit, and reintroduced after rejuvenation at the top of the zone from where it was drawn, and whereby said catalyst is sequentially sent into the regeneration circuit,
whereby the feedstock of a reaction zone of row n consists of effluents of the reaction zone of row n-1, to which are added a fraction of the olefinic feedstock, and whereby the aromatic compound that is to be alkylated is introduced at the input of the first reaction zone.

17. Process for the production according to claim 16 that consists of a series of n reaction zones, whereby n is between 1 and 5.

## Patentansprüche

1. Verfahren zur Herstellung von Phenylalkanen durch Alkylierung einer aromatischen Verbindung durch eine Beschickung, die C9-C16-Olefine enthält, bei dem mindestens ein Reaktor eingesetzt wird, der in mehrere Reaktionszonen aufgeteilt ist, wobei jede Reaktionszone einen Festsäurekatalysator verwendet, der im Bewegtbett arbeitet, wobei der Katalysator jeder Reaktionszone bei seinem Austritt aus der Reaktionszone auf kontinuierliche Weise in einen Aktivierungskreislauf geschickt wird, dann am Kopf der Zone wieder eingeführt wird, und wobei der Katalysator jeder Reaktionszone am Ausgang der Reaktionszone auf sequentielle Weise in einen Regenerationskreislauf geschickt, dann am Kopf der Reaktionszone wieder eingeführt wird.

2. Verfahren zur Herstellung von Phenylalkanen durch Alkylierung einer aromatischen Verbindung durch eine Beschickung, die C9-C16-Olefine enthält, nach Anspruch 1, wobei ein Teil der Reaktionszonen eine untergeordnete Reihe bildet, die mit dem gleichen Katalysator arbeitet, der sie im Bewegtbett nacheinander durchläuft, wobei der Katalysator auf kontinuierliche Weise in den Aktivierungskreislauf am Ausgang der letzten Zone der untergeordneten Reihe eingeführt wird, dann nach der Aktivierung am Kopf der ersten Zone der untergeordnete Serie wieder eingeführt wird, wobei der Katalysator auf sequentielle Weise in den Regenerationskreislauf am Ausgang der letzten Zone der untergeordneten Reihe geschickt wird und nach der Regeneration am Kopf der ersten Zone der untergeordneten Reihe wieder eingeführt wird.

3. Verfahren zur Herstellung von Phenylalkanen durch Alkylierung nach irgendeinem der Ansprüche 1 bis 2,
wobei die olefinische Alkylierungsbeschickung bevorzugt Olefine enthält, die 10 bis 14 C-Atome aufweisen, und die zu alkylierende aromatische Verbindung Benzol ist.

4. Verfahren zur Herstellung von Phenylalkanen durch Alkylierung nach irgendeinem der Ansprüche 1 bis 3,
wobei die Olefinbeschickung auf fraktionierte Weise in jede Reaktionszone eingeführt wird, so dass das Verhältnis aromatische Verbindung/Olefine am Eingang jeder Reaktionszone im Bereich zwischen 30 und 100, und bevorzugt im Bereich zwischen 30 und 70 liegt.

5. Verfahren zur Herstellung von Phenylalkanen durch Alkylierung einer aromatischen Verbindung nach irgendeinem der Ansprüche 1 bis 4, wobei der verwendete Katalysator mindestens in einem Teil der Reaktionszonen ein kristallisierter, fester Säurekatalysator des Strukturtyps ist, der aus der Gruppe, gebildet aus FAU, MOR, MTW, OFF, MAZ, BEA, EUO, UTL und NES ausgewählt ist.

6. Verfahren zur Herstellung von Phenylalkanen durch Alkylierung einer aromatischen Verbindung nach irgendeinem der Ansprüche 1 bis 4, wobei der verwendete Katalysator in mindestens einem Teil der Reaktionszonen ein amorpher fester Katalysator ist, der aus der Gruppe, gebildet aus den Siliciumdioxid-Aluminiumoxiden und den mit Halogenverbindungen dotieren oder nicht dotierten Aluminiumoxiden ausgewählt ist.

7. Verfahren zur Herstellung von Phenylalkanen durch Alkylierung einer aromatischen Verbindung nach irgendeinem der Ansprüche 1 bis 5, wobei der in bestimmten Reaktionszonen verwendete Katalysator ein entaluminierter Y-Zeolith mit einem Si/Al-Verhältnis im Bereich zwischen 8 und 100 und bevorzugt im Bereich zwischen 15 und 70 ist.

8. Verfahren zur Herstellung von Phenylalkanen durch Alkylierung einer aromatischen Verbindung nach irgendeinem der Ansprüche 1 bis 7, wobei die Größe der Katalysatorkörner, die in jeder der Reaktionszonen verwendete werden, im Bereich zwischen 0,5 und 5 mm und bevorzugt im Bereich zwischen 1 und 4 mm liegt.

9. Verfahren zur Herstellung von Phenylalkanen durch Alkylierung einer aromatischen Verbindung nach irgendeinem der Ansprüche 1 bis 8, wobei der in bestimmten Reaktionszonen verwendete Katalysator ein Gemisch aus mindestens zwei kristallisierten festen Katalysatoren des Strukturtyps ist, der aus der Gruppe, gebildet aus FAU, MOR, MTW, OFF, MAZ, BEA, EUO, UTL und NES, ausgewählt ist.

10. Verfahren zur Herstellung von Phenylalkanen durch Alkylierung einer aromatischen Verbindung nach irgendeinem der Ansprüche 1 bis 8, wobei der in bestimmten Reaktionszonen verwendete Katalysator ein Gemisch aus Zeolithen ist, wobei einer der Bestandteile des Gemischs ein entaluminierter Y-Zeolith mit einem Si/Al-Verhältnis im Bereich zwischen 8 und 100 und bevorzugt im Bereich zwischen 15 und 70 ist, und der andere Bestandteil des Gemischs ein Zeolith des Strukturtyps MOR, bevorzugt Mordenit, ist.

11. Verfahren zur Herstellung von Phenylalkanen durch Alkylierung einer aromatischen Verbindung nach irgendeinem der Ansprüche 1 bis 10, wobei der in bestimmten Reaktionszonen verwendete Katalysator ein Gemisch aus mindestens einem kristallisierten festen Katalysator des Strukturtyps ist, der aus der Gruppe, gebildet aus FAU, MOR, MTW, OFF, MAZ, BEA, EUO, UTL, NES ausgewählt ist, und einem amorphen festen Katalysator, der aus der Gruppe, gebildet aus den Siliciumdioxid-Aluminiumoxiden und den Aluminiumoxiden, die mit Halogenverbindungen dotiert sind oder nicht dotiert sind, ausgewählt ist.

12. Verfahren zur Herstellung von Phenylalkanen durch Alkylierung einer aromatischen Verbindung nach irgendeinem der Ansprüche 1 bis 11, wobei der Gehalt an schweren aromatischen Verbindungen, die in der olefinischen Alkylierungsbeschickung enthalten sind, bis zu 80 Ges.-% reichen kann, wobei sich dieser Prozentsatz nur auf die Olefine und die die Aromaten bezieht.

13. Verfahren zur Herstellung von Phenylalkanen durch Alkylierung einer aromatischen Verbindung nach irgendeinem der Ansprüche 1 bis 12, wobei die Alkylierungstemperatur im Bereich zwischen 50 °C und 350 °C liegt und der Druck im Bereich zwischen 1 und 10 MPa liegt.

14. Verfahren zur Herstellung von Phenylalkanen durch Alkylierung einer aromatischen Verbindung nach irgendeinem der Ansprüche 1 bis 13, wobei das Verbrennungsgas, das für die Regeneration des Katalysators verwendet wird, 0,1 bis 20 Vol-% Sauerstoff enthält, und die Verbrennungstemperatur im Bereich zwischen 300 °C und 600 °C liegt.

15. Verfahren zur Herstellung von Phenylalkanen durch Alkylierung einer aromatischen Verbindung nach irgendeinem der Ansprüche 1 bis 14, wobei das Aktivierungsmittel eine aromatische Verbindung, bevorzugt Benzol ist, und die Aktivierungstemperatur größer als 100 °C ist.

16. Reaktor zur Herstellung von Phenylalkanen nach Anspruch 1 durch Alkylierung einer aromatischen Verbindung durch eine Olefinbeschickung, der aus einer Reihe von n Reaktionszonen besteht, wobei n im Bereich zwischen 1 und 10 liegt, und die Reihe Folgendes umfasst:
- eine erste Untergruppe von Reaktionszonen, die nacheinander von dem Katalysator, der im Bewegtbett zirkuliert, durchlaufen wird, wobei der am Ausgang der letzten Zone der ersten Untergruppe entnommene Katalysator kontinuierlich in den Aktivierungskreislauf eingeführt und am Kopf der ersten Zone des ersten Untergruppe wieder eingeführt wird, und wobei der Katalysator auf sequentiell Weise in den Regenerationskreislauf geschickt wird,
- eine zweite Untergruppe, die aus Reaktionszonen besteht, die mit einem Katalysator arbeiten, der im Bewegtbett arbeitet, wobei jede Reaktionszone einen eigenen Aktivierungskreislauf besitzt, wobei der Katalysator am Ausgang jeder der Zonen entnommen, kontinuierlich in den Aktivierungskreislauf eingeführt und nach der Aktivierung am Kopf der Zone wieder eingeführt wird, aus der er entnommen wurde, und wobei der Katalysator auf sequentielle Weise in den Regenerationskreislauf geschickt wird,
wobei die Beschickung einer Reaktionszone mit Rangstufe n aus Abflüssen aus der Reaktionszone mit Rangstufe n-1 besteht, zu der eine Fraktion der Olefinbeschickung hinzukommt, und wobei die zu alkylierende aromatische Verbindung am Eingang der ersten Reaktionszone eingeführt wird.

17. Reaktor zur Herstellung nach Anspruch 16, wobei die Anzahl von n Reaktionszonen im Bereich zwischen 1 und 5 liegt.
